(19) Europäisches Patentamt
European Patent Office
Office européen des brevets

(11) Publication number: 0 082 699 B1

(12) # EUROPEAN PATENT SPECIFICATION

(45) Date of publication of patent specification: 13.11.85

(51) Int. Cl.⁴: C 07 D 241/44, A 01 N 43/60

(21) Application number: 82306769.9

(22) Date of filing: 17.12.82

(54) Herbicidal quinoxalinyl ethers.

(30) Priority: 17.12.81 US 331685

(43) Date of publication of application: 29.06.83 Bulletin 83/26

(45) Publication of the grant of the patent: 13.11.85 Bulletin 85/46

(84) Designated Contracting States: BE DE FR NL

(56) References cited:
EP-A-0 023 785
EP-A-0 042 750
DE-A-3 004 770
GB-A-2 042 539

(73) Proprietor: E.I. DU PONT DE NEMOURS AND COMPANY
1007 Market Street
Wilmington Delaware 19898 (US)

(72) Inventor: Fawzi, Maged Mohamed
26 Glenbarry Drive
Wilmington Delaware 19808 (US)

(74) Representative: Hildyard, Edward Martin et al
Frank B. Dehn & Co. Imperial House 15-19 Kingsway
London WC2B 6UZ (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 082 699 B1

Courier Press, Leamington Spa, England.

**Description**

Background of the invention

This invention relates to quinoxalinyl ethers which are useful as herbicides. These compounds are especially useful as selective weed control agents in various broadleaf crops and in paddy rice, providing excellent control of barnyardgrass (*Echinochloa* spp).

DE—A—30 04 770 discloses quinoxaline and quinoline compounds of the formula

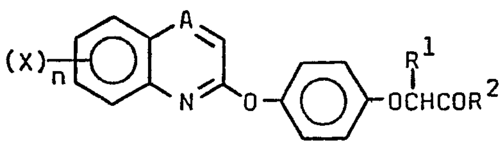

wherein

A is CH or N;

x is halogen;

n is 0, 1 or 2;

$R^1$ is H or lower alkyl;

$R^2$ is OH, alkoxy, OM, $NR^3R^4$, lower alkenyloxy, benzyloxy, alkoxyalkoxy, phenoxy, cyclohexyloxy, haloalkoxy, alkynyloxy or cyanoalkoxy;

M is an organic or inorganic cation;

$R^3$ is H or lower alkyl; and

$R^4$ is H or lower alkyl.

EP—A—23,785 discloses herbicidal quinoxaline compounds of the general formula:

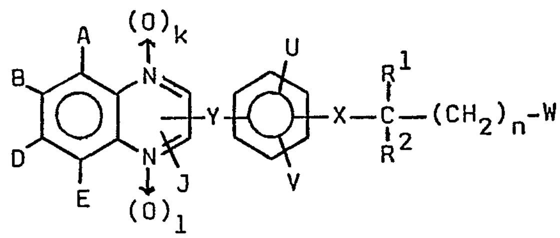

where

A, B, D, E, J, U and V may be halogen, among others;

$R^1$ and $R^2$ may be H or $C_1$—$C_6$ alkyl, among others;

W may be COG and G may be $C_1$—$C_{10}$ alkoxy substituted with $C_1C_6$ alkoxy.

Neither of these publications disclose the ethoxyethoxyethyl and methoxyethoxyethyl compounds disclosed and claimed herein.

Our EP—A—42,750, unpublished at the priority date of the present Application, discloses a mixture of 2-ethoxyethoxyethyl 2-[4-(6-chloroquinoxalin-2-yloxy)phenoxy]propanoate with the corresponding 7-chloro isomer. The mixture showed selective herbicial activity.

Summary of the invention

It has been found that the novel compounds of Formula (I) are useful as herbicides, especially for controlling weeds such as barnyardgrass

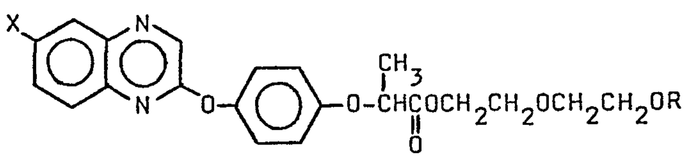

(I)

where

X is Cl, F or Br; and

R is $CH_3$ or $CH_2CH_3$.

Specifically preferred for its outstanding utility is the compound 2-ethoxyethoxyethyl-2-[4 - (6 - chloroquinoxalin - 2 - yloxy)phenoxy]propanoate.

This invention therefore relates to compounds of Formula I, herbicidal compositions containing them and methods of using them to control weeds. It should be noted that the 6-haloquinoxaline derivatives of the present invention exhibit for superior herbicidal activity to the 6 or 7-haloquinoxaline isomer mixtures disclosed in our EP—A—42,750. For example, the activity of 2-ethoxyethoxyethyl - 2 - [4 - 6 - chloroquinoxalin - 2 - yloxy)phenoxy]propanoate for pre-emergence control of weeds such as bluegrass, dallisgrass, giant foxtail and wild oats is about twice that of the 6 or 7-chloro isomeric mixture.

Moreover the compounds of formula (I) in general are surprisingly effective herbicides by comparison

with some compounds of closely related structure. For example, the selectivity of these compounds to paddy rice at rates of application which give excellent control of *Echinochloa* spp. is a valuable and unexpected feature.

Detailed description

Synthesis

The various compounds of Formula I can be prepared by one of the following methods.

Method A

The compounds (I) can be prepared by combining, preferably in equimolar amounts, a 2-chloro-6-haloquinoxaline (II) and the alkali metal salt of methoxy or ethoxyethoxyethyl 2-(4-hydroxy-phenoxy)propanoate (III) as illustrated below:

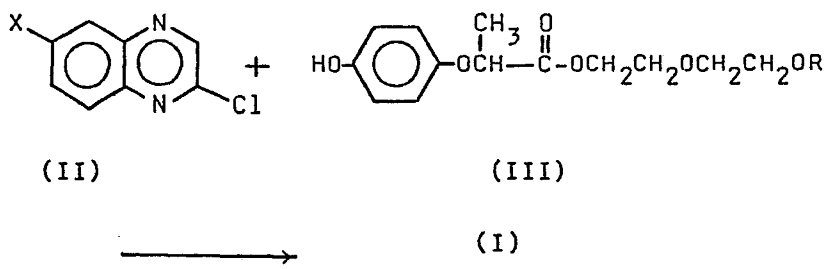

(II) (III)

(I)

where

X = Cl, F or Br; and

R = $CH_3$ or $C_2H_5$.

Suitable solvents for the reaction include methyl ethyl ketone, dimethylformamide, dimethylsulfoxide and diglyme. The reaction is preferably carried out at temperatures between 25 and about 130°C.

The 2-chloro-6-haloquinoxalines (II) used in this invention can be prepared by methods known in the art. Glyoxylic acid or an alkyl ester of glyoxylic acid is combined with a halosubstituted *o*-phenylenediamine in ethanol, to yield a mixture of 6- and 7-halo-2-hydroxyquinoxalines, as described in *J.A.C.S. 71*, 6, (1949).

The mixture of the 2-hydroxyquinoxalines is treated with phosphorous oxychloride as described in *J. Chem. Soc.*, 519 (1948) or *Bull. Soc. Chem. France*, 356 (1963), to give the desired 2-chloro-6-haloquinoxaline and its 7-isomer. The reader is referred to the above cited references for further information. The two isomers can be separated at this stage by methods known in the art such as chromatography or crystallization.

The methoxy or ethoxyethoxyethyl 2-(4-hydroxyphenoxy)propionates (III) can be prepared by the alkylation of 4-benzyloxyphenol with the alkyl bromopropionate, followed by catalytic hydrogenation of the product in the presence of palladium over carbon to yield the desired product (III).

Method B

The esters (I) can also be synthesized by the reaction of the appropriate acid chloride (IV) with 2 - (2 - methoxyethoxy)ethanol or with 2 - (2 - ethoxyethoxy)ethanol in a suitable solvent such as tetrahydrofuran in the presence of an acid acceptor as outlined below:

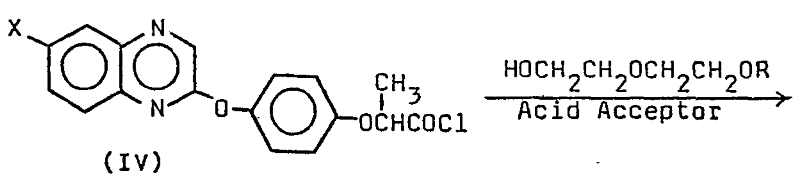


(I)

(IV)

The requisite acid chlorides (IV) can be prepared via the acid from the corresponding methyl or ethyl 2 - (4 - (6 - halo - 2 - quinoxalyloxy)phenoxy]propanoates by methods that are known in the art.

The methyl and ethyl 2-[4-(6-halo-2-quinoxalinyloxy)phenoxy]propanoates can be prepared by the procedure described under Method A, using methyl or ethyl 2 - (4 - hydroxyphenoxy)propanoate in lieu of (III).

Method C

The condensation of the alkali metal salt of a 6-halo-2-(4-hydroxyphenoxy)quinoxaline (V) with methoxy or ethoxyethoxyethyl 2-bromopropanoate (VI) in a suitable solvent such as methyl ethyl ketone or DMF provides another synthetic procedure that may be used to prepare the compounds (I) disclosed herein.

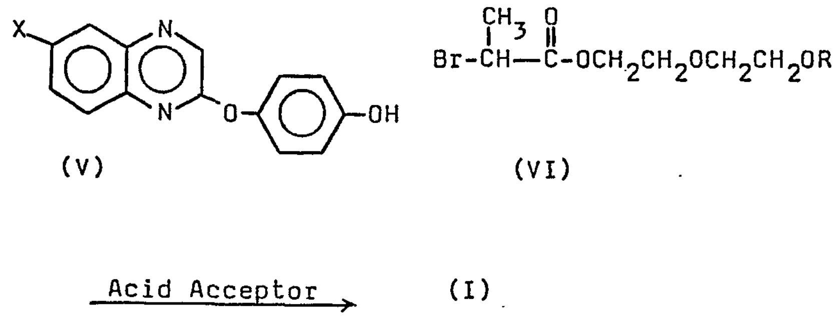


(V) (VI)

(I)

The 6-halo-2-(4-hydroxyphenoxy)quinoxalines (V) are prepared by the multi-step synthesis outlined below:

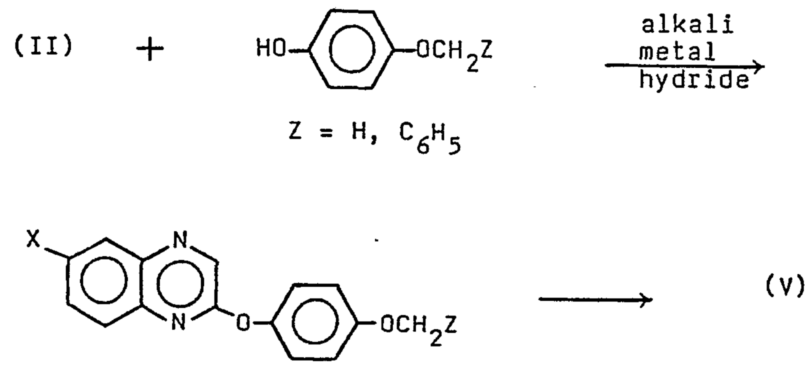


(II) +

Z = H, $C_6H_5$

(VII) (V)

A 2-chloro-6-haloquinoxaline is condensed with 4-benxyloxyphenol or 4-methoxyphenol in the presence of sodium methoxide or an alkali metal hydride to give (VII). Next the methyl or benzyl group is removed by methods known in the art, for example, those described in Belgian Patent 868,875 or Tetrahedron 24, 2289 (1968), to both of which the reader is referred for further information, to yield the desired 6-halo-2-(4-hydroxyphenoxy)quinoxalines.

Esters of type (VI) are readily prepared by the addition of 2-bromopropionyl chloride to methoxy or ethoxyethoxyethanol in the presence of an acid acceptor.

The following examples illustrate the preparation of some of the compounds of the invention.

Example 1

2-Methoxyethoxyethyl 2[4-(6-chloro-2-quinoxalinyloxy)phenoxy]propanoate

In a nitrogen atmosphere, slowly and a solution of 5.7 g (0.02 mole) methoxyethoxyethyl 2 - (4 - hydroxyphenoxy)propanoate in 25 cc of dimethylformamide to 0.5 g (0.02 mole) sodium hydride in 25 cc of DMF. When the evolution of hydrogen ceases, stir at room temperature for 30 minutes, then add 4.0 g (0.02 mole) 2,6-dichloroquinoxaline. Heat at 130°C for 6 hours and then cool to room temperature. Pour into about 200 cc of ice cold water, and extract the product into methylene chloride. Wash the $CH_2Cl_2$ extract with water, dry and concentrate under vacuum to yield the desired ester.

Following the teachings of Example 1 and by substituting an appropriate 2-chloroquinoxaline and an appropriate alkyl 2 - (4 - hydroxyphenoxy)propanoate, the esters in Table I can be prepared.

TABLE I

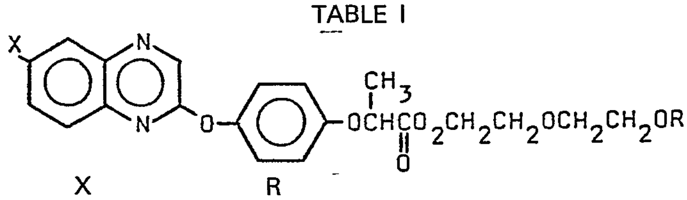

| X | R | |
|---|---|---|
| F | $CH_3$ | |
| Br | $CH_3$ | |
| Cl | $C_2H_5$ | $n_D^{24}=1.5655$ |
| F | $C_2H_5$ | |
| Br | $C_2H_5$ | |

Example 2

2-Ethoxyethoxyethyl 2-[4-(6-chloro-2-quinoxalinyloxy)phenoxy]propanoate

The title compound was prepared by treatment of 2-[4-(6-chloro-2-quinoxalinyloxy)phenoxy]propanoic acid in chlorobutane with thionyl chloride and a catalytic amount of dimethylformamide. To a cold (5°C) solution of 3.15 g (.009 mol) 2-[4-(6-chloro - 2-quinoxalinyloxy)phenoxy]propionyl chloride in 50 cc tetrahydrofuran, was added a solution of 0.7 cc pyridine and 1.35 cc (0.009 mol) 2 - (2 - ethoxyethoxy)ethanol in 5 cc tetrahydrofuran. The reaction mixture was stirred overnight at room temperature. Insoluble material was removed by filtration. The filtrate was concentrated under vacuum, dissolved in methylene chloride, washed with 5% hydrochloric acid and 50% sodium bicarbonate solutions, dried over magnesium sulfate, and concentrated under vacuum to give 2.2 g of the product, $n_D^{24}$=1.5655.

NMR ($CDCl_3$)δ: 1.2 (t, J=7 Hz, 3H);
1.7 (d, J=7 Hz, 3H);
3.6 (m, 8H);
4.3 (m, 2H);
4.8 (q, J=7 Hz, 1H);
7.1 (m, 4H);
7.6 (m, 2H);
8.1 (m, 1H);
8.7 (s, 1H).

The procedure outlined above can also be used to prepare the compounds in Table II.

TABLE II

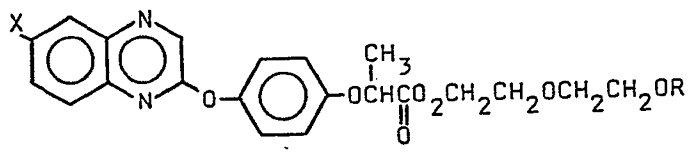

| X | R |
|---|---|
| F | $CH_3$ |
| Br | $CH_3$ |
| Cl | $CH_3$ |
| F | $C_2H_5$ |
| Br | $C_2H_5$ |

Example 3

2-Methoxyethoxyethyl 2-[4-(6-fluoro-2-quinoxalinyloxy)phenoxy]propanoate

In a nitrogen atmosphere, add a solution of 5.1 g (0.02 mole) 6-fluoro-2-(4-hydroxyphenoxy)quinoxaline in 30 cc dimethylformamide to 0.9 g (0.02 mole) 57% sodium hydride in 20 cc dimethylformamide at about 20°C. When the evolution of hydrogen ceases, add 5.1 g (0.02 mole) methoxyethoxyethoxyethyl 2-bromopropanoate. Heat the reaction mixture at approximately 80°C until the reaction is complete. Pour the reaction mixture into water and extract with methylene chloride. Combine the ethereal extracts and dry over magnesium sulfate. Removal of the methylene chloride gives the desired product.

The compounds listed in Table III can be prepared by a similar fashion from the appropriate 2 - (4 - hydroxyphenoxy)quinoxaline and the methoxy or ethoxyethoxyethyl 2-bromopropionate.

TABLE III

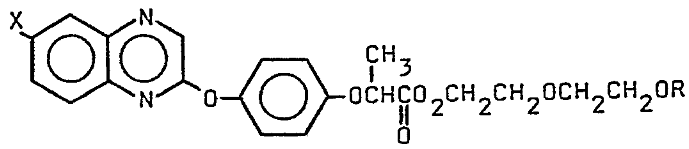

| X | R | |
|---|---|---|
| Cl | $CH_3$ | |
| Br | $CH_3$ | |
| Cl | $C_2H_5$ | $n_D^{24}=1.5655$ |
| F | $C_2H_5$ | |
| Br | $C_2H_5$ | |

Formulations

Useful formulations of the compounds of Formula I can be prepared in conventional ways. They include dusts, granules, pellets, wettable powders, emulsifiable concentrates and the like. Many of these may be applied directly. Sprayable formulations can be extended in suitable media and used at spray volumes of from a few liters to several hundred liters per hectare. High strength compositions are primarily used as intermediates for further formulation. The formulations, broadly, contain about 0.1% in 99% by weight of active ingredient(s) and at least one of (a) about 0.1% to 20% surfactant(s) and (b) about 1% to 99.9% solid or liquid diluent(s). More specifically, they will contain these ingredients in the following approximate proportions:

TABLE IV

| | Active ingredient | Weight percent* Diluent(s) | Surfactant(s) |
|---|---|---|---|
| Wettable Powders | 5—25 | 65—94 | 1—10 |
| Emulsifiable Concentrates | 3—50 | 35—97 | 0—15 |
| Dusts | 1—25 | 70—99 | 0—5 |
| Granules and Pellets | 0.1—20 | 75—99.9 | 0—15 |
| High Strength Compositions | 90—99 | 0—10 | 0—2 |

*Active ingredient plus at least one of a Surfactant or a Diluent equals 100 weight percent.

Lower or higher levels of active ingredient can, of course, be present depending on the intended use and the physical properties of the compound. Higher ratios of surfactant to active ingredient are sometimes desirable, and are achieved by incorporation into the formulation or by tank mixing.

Typical solid diluents are described in Watkins, et al., "Handbood of Insecticide Dust Diluents and Carriers", 2nd Ed., Dorland Books, Caldwell, New Jersey, but other solids, either mined or manufactured, may be used. The more absorptive diluents are preferred for wettable powders and the denser ones for dusts. Typical liquid diluents and solvents are described in Marsden, "Solvents Guide", 2nd Ed., Interscience, New York, 1950. Solubility under 0.1% is preferred for suspension concentrates; solution concentrates are preferably stable against phase separation at 0°C. "McCutcheon's Detergents and Emulsifiers Annual", MC Publishing Corp., Ridgewood, New Jersey, as well as Sisely and Wood, "Encyclopedia of Surface Active Agents", Chemical Publishing Co., Inc., New York, 1964, list surfactants and recommended uses. All formulations can contain minor amounts of additives to reduce foaming, caking, corrosion, microbiological growth, etc.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, Littler, U.S. Patent 3,060,084). Granules and pellets may be made by spraying the active material upon preformed granular carriers or by agglomeration techniques. See J. E. Browning, "Agglomeration", *Chemical Engineering*, December 4, 1967, pp. 147ff. and "Perry's Chemical Engineer's Handbook", 5th Ed., McGraw-Hill, New York, 1973, pp. 8—57ff.

For further information regarding the art of formulation, see for example:

H. M. Loux, U.S. Patent 3,235,361, February 15, 1966, Col. 6, line 16 through Col. 7, line 19 and Examples 10 through 41;

R. W. Luckenbaugh, U.S. Patent 3,309,192, March 14, 1967, Col. 5, line 43 through Col. 7, line 62 and Examples 8, 12, 15, 39, 41, 52, 53, 58, 132, 138—140, 162—164, 166, 167 and 169—182;

H. Gysin and E. Knusli, U.S. Patent 2,891,855, June 23, 1959, Col. 3, line 66 through Col. 5, line 17 and Examples 1—4;

G. C. Klingman, "Weed Control as a Science", John Wiley & Sons, Inc., New York, 1961, pp. 81—96; and

J. D. Fryer and S. A. Evans, "Weed Control Handbook", 5th Ed., Blackwell Scientific Publications, Oxford, 1968, pp. 101—103.

In the following examples, all parts are by weight unless otherwise indicated.

Example 4
Wettable powder

| | |
|---|---|
| 2-methoxyethoxyethyl 2-[4-(6-bromoquinoxalin-2-yl)-oxy)phenoxy]propanoate | 5% |
| sodium alkylnaphthalenesulfonate | 2% |
| sodium ligninsulfonate | 2% |
| synthetic amorphous silica | 3% |
| kaolinite | 88% |

The ingredients are blended, ball-milled, passed through 50 mesh screen (0.3 mm openings), reblended, and packaged.

Example 5
Wettable powder

| | |
|---|---|
| 2-ethoxyethoxyethyl 2-[4-(6-chloroquinoxalin-2-yl-oxy)phenoxy]propanoate | 25% |
| sodium alkylnaphthalenesulfonate | 2% |
| low viscosity methyl cellulose | 2% |
| diatomaceous earth | 71% |

The active ingredient is dissolved in a suitable solvent and sprayed on the blended mixture of surfactants and diluents in a rotary container. After solvent evaporation, the blended mixture is ball-milled and passed through a 50 mesh screen 0.3 mm openings. The product is reblended before packaging.

Example 6
Granule

| | |
|---|---|
| Wettable Powder of Example 5 | 5% |
| attapulgite granules (U.S.S. 20—40 mesh; 0.84—0.42 mm) | 95% |

A slurry of wettable powder containing ≈25% solids is sprayed on the surface of attapulgite granules in a double-cone blender. The granules are dried and packaged.

Example 7
Extruded pellet

| | |
|---|---|
| 2-ethoxyethoxyethyl 2-[4-(6-chloroquinoxalin-2-yl-oxy)phenoxy]propanoate | 10% |
| anhydrous sodium sulfate | 10% |
| crude calcium ligninsulfonate | 5% |
| sodium alkylnaphthalenesulfonate | 1% |
| calcium/magnesium bentonite | 74% |

The ingredients are blended, hammer-milled and then moistened with about 12% water. The mixture is extruded as cylinders about 3 mm diameter which are cut to produce pellets about 3 mm long. These

may be used directly after drying, or the dried pellets may be crushed to pass a U.S.S. No. 20 sieve (0.84 mm openings). The granules held on a U.S.S. No. 40 sieve (0.42 mm openings) may be packaged for use and the fines recycled.

Example 8
Wettable powder

| | |
|---|---|
| 2-methoxyethoxyethyl 2-[4-(6-fluoroquinoxalin-2-yl-oxy)phenoxy]propanoate | 20% |
| sodium alkylnaphthalenesulfonate | 4% |
| sodium ligninsulfonate | 4% |
| low viscosity methyl cellulose | 3% |
| attapulgite "A" | 69% |

The ingredients are thoroughly blended. After grinding in a hammer-mill to produce particles essentially all below 100 microns, the material is reblended and sifted through a U.S.S. No. 50 sieve (0.3 mm opening) and packaged.

Example 9
Low strength granule

| | |
|---|---|
| 2-ethoxyethoxyethyl 2-[4-(6-fluoroquinoxalin-2-yl-oxy)phenoxy]propanoate | 1% |
| N,N-dimethylformamide | 9% |
| attapulgite granules (U.S.S. 20—40 sieve; 0.84—0.42 mm openings) | 90% |

The active ingredient is dissolved in the solvent and the solution is sprayed upon dedusted granules in a double cone blender. After spraying of the solution has been completed, the blender is allowed to run for a short period and then the granules are packaged.

Example 10
Low strength granule

| | |
|---|---|
| 2-methoxyethoxyethyl 2-[4-(6-chloroquinoxalin-2-yl-oxy)phenoxy]propanoate | 0.1% |
| attapulgite granules (U.S.S. 20—40 mesh); 0.84—0.42 mm openings) | 99.9% |

The active ingredient is dissolved in a solvent and the solution is sprayed upon dedusted granules in a double-cone blender. After spraying of the solution has been completed, the material is warmed to evaporate the solvent. The material is allowed to cool and then packaged.

Example 11
Granule

| | |
|---|---|
| 2-ethoxyethoxyethyl 2-[4-(6-chloroquinoxalin-2-yl-oxy)phenoxy]propanoate | 20% |
| wetting agent | 1% |
| crude ligninsulfonate salt (containing 5—20% of the natural sugars) | 10% |
| attapulgite clay | 69% |

The ingredients are blended and milled to pass through a 100 mesh screen. This material is then added to a fluid bed granulator, the air flow is adjusted to gently fluidize the material, and a fine spray of water is sprayed onto the fluidized material. The fluidization and spraying are continued until granules of the desired size range are made. The spraying is stopped, but fluidization is continued, optionally with heat, until the water content is reduced to the desired level, generally less than 1%. The material is then discharged, screened to the desired size range, generally 14—100 mesh (1410—149 microns), and packaged for use.

Example 12
High strength concentrate

| | |
|---|---|
| 2-ethoxyethoxyethyl 2-[4-(6-chloroquinoxalin-2-yl-oxy)phenoxy)propanoate | 99% |
| dodecylphenol polyethylene glycol ether | 1.0% |

The ingredients are blended and bottled in a suitable container. The concentrate may be formulated further if necessary.

Example 13
Dust

| | |
|---|---|
| 2-ethoxyethoxyethyl 2-[4-(6-bromoquinoxalin-2-yl-oxy)phenoxy]propanoate | 10% |
| synthetic silica | 10% |
| Pyrophyllite | 80% |

The active ingredient is blended with synthetic silica and then passed through a hammer-mill to produce particles substantially all below 200 microns. The ground concentrate is then blended with powdered pyrophyllite until homogeneous.

Example 14
Emulsifiable concentrate

| | |
|---|---|
| 2-ethoxyethoxyethyl 2-[4-(6-chloroquinoxalin-2-yl-oxy)phenoxy]propanoate | 20% |
| chlorobenzene | 74% |
| sorbitan monostearate and polyoxyethylene condensates thereof | 6% |

The ingredients are combined and stirred to produce a solution which can be emulsified in water for application.

Utility

The compounds of the present invention are useful for broad-spectrum weed control and for controlling grass weeds in broadleaf crops such as beans, flax, sugarbeet, cabbage, tomatoes, potatoes, carrots, cucurbits, endive, beets, etc: Suitable application rates will generally be found in the range from 0.005 to 20 hg/ha, preferably 0.02 to 10 kg/ha, the lower rates being selected for use in the presence of crop plants. The utility of these compounds is broadly similar to those of our EP—A—42,750, to which the reader is referred for further information. The compounds also have a particular and unexpected utility on rice.

Rice is one of the world's most important crops and is the main food source for much of the world's population. In fact, on an acreage basis, rice ranks as the second largest crop in the entire world. It is very important that the yield of rice be maximized to feed a hungry world. Accordingly, it is important that weeds that compete with rice for available light, nutrients, etc., be controlled so as to produce rice in a high yield.

Barnyardgrass (*Echinochloa crusgalli*), and closely related Echinochloa plants are extremely prevelant and harmful weeds in rice plantings around the world. Indeed, the well-known journal "Agrichemical Age" (May, 1977 issue, page 11) reported that a barnyardgrass infestation of only one plant per square foot (10.76 plants per $m^2$), if allowed to compete all season with a good stand of rice, would reduce the rice yield by 25%. Yet, a normal infestation of barnyardgrass in a rice stand can be many times as dense. Therefore it is obvious that barnyardgrass can severely reduce the yield or rice, and compounds that selectively control barnyardgrass in rice plantings are extremely valuable to mankind. The present invention teaches that certain novel compounds of Formula I have the important utility of selectively controlling barnyardgrass in rice plantings.

To control the growth of weeds in paddy rice, compounds of Formula I are applied to the paddy at a rate of about 0.5 to 50 grams per hectare, preferably about 2 to 20 grams per hectare, the application rate depending on considerations such as the soil type, weed pressure, timing of application, and particular compound applied. The compounds can be applied in several ways. They can be applied directly to the water surface of the rice paddy either about one to four days before transplanting rice into the paddy or about two to twenty days after transplanting rice into the paddy. Alternatively, they can be applied directly to the paddy soil before flooding and transplanting. For reasons of effectiveness and increased rice tolerance, the compounds are preferably applied between about -three to twelve days after transplanting.

The following example demonstrates the utility of the compounds of the present invention in comparison with a closely related compound within the scope of a prior disclosure (Compound A, disclosed generically in DE—A—3,004,770 and EP—A—23,785 but not prepared or characterised).

Example 15

Test samples were formulated in a non-phytotoxic solvent and applied to the paddy water three days after transplanting of rice. The paddy soil contained propagules of several weeds which were just beginning to sprout. The test was maintained in a greenhouse, and plant response ratings were taken six weeks after application. The plant response ratings are presented in Table III and consist of a number and a letter. The number ranges from zero to ten and represents the extent of the response, with zero indicating no response and ten indicating 100% control. The letter describes the type of the response, with "C" representing chlorosis/necrosis (chronic response) and "G" representing growth retardation.

TABLE III

(A)

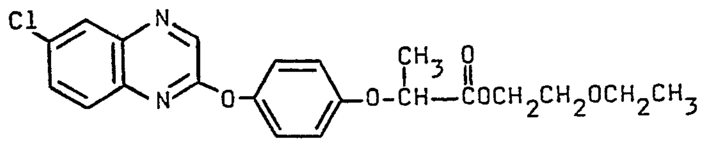

(B)

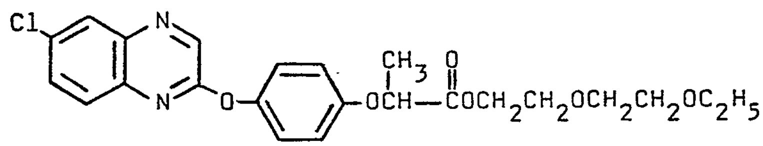

| Treatment | Rate, g ai/ha | Rice | Barnyard-grass* | Water chestnut* |
|---|---|---|---|---|
| A | 1 | 0 | 0 | 0 |
| | 4 | 0 | 5C | — |
| | 8 | 0 | 9C | 0 |
| B | 1 | 0 | 9C | 0 |
| | 4 | 1G,2C | 10C | 0 |
| | 8 | 9C | 10C | 10C |

**Echinochloa* sp. and *Eleocharis* sp., respectively.

In reviewing the data presented in Table III it is evident that the compound of this invention, Compound B, is far more effective in controlling barnyardgrass than the art compound, Compound A.

**Claims**

1. A compound of the formula:

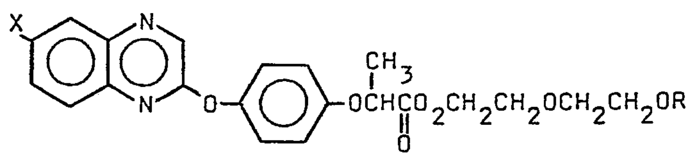

where

X is Cl, F or Br; and

R is $CH_3$ or $CH_2CH_3$;

excluding mixtures of 2-ethoxyethoxyethyl-2-[4-(6-chloroquinoxalin-2-yloxy)phenoxy]propanoate with a substantial proportion of the corresponding 7-chloro isomer.

2. The compound of Claim 1, 2-ethoxyethoxyethyl-2-[4-(6-chloroquinoxalin-2-yloxy)phenoxy]propanoate, excluding mixtures thereof with a substantial proportion of the corresponding 7-chloro isomer.

3. The compound of Claim 1, 2-methoxyethoxyethyl 2-[4-(6-chloro-2-quinoxalinyloxy)phenoxy]propanoate.

4. The compound of Claim 1, 2-methoxyethoxyethyl 2-[4-(6-fluoro-2-quinoxalinyloxy)phenoxy]propanoate.

5. A composition for the control of undesirable vegetation comprising an effective amount of a herbicidal compound and at least one of (a) a surface active agent and (b) a solid or liquid diluent, characterised in that said herbicidal compound comprises a compound of any of claims 1 to 4.

6. A method for the selective control of undesirable vegetation in the presence of crop plants by applying to the locus to be protected an effective amount of a herbicidal compound, characterised in that said herbicidal compound comprises a compound of any of claims 1 to 4.

7. The method of claim 6 wherein said crop plants are paddy rice.

8. The method of claim 7 wherein said compound is applied at a rate of from 2 to 20 g/ha.

9. The method of claim 6, 7 or 8 wherein the compound of claim 2 is applied.

10. A process for the preparation of a compound of claim 1 which comprises:

(a) contacting an alkali metal salt of a compound of formula

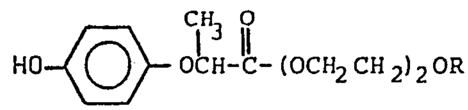

with a 2-chloro-6-haloquinoxaline of formula

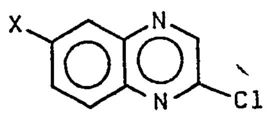

wherein R and X are as defined in claim 1; or

(b) reacting an acid chloride of formula

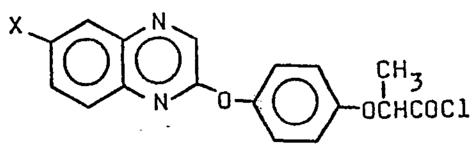

wherein X is as defined in claim 1, with 2-(2-methoxyethoxy)ethanol or 2-(2-ethoxyethoxy)ethanol in the presence of an acid acceptor; or

(c) reacting a 6-halo-2-(4-hydroxyphenoxy)quinoxaline of formula

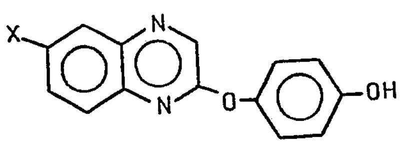

with a bromoester of formula

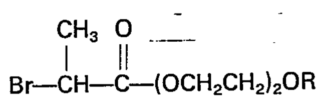

in the presence of an acid acceptor, wherein X and R are as defined in claim 1.

**Patentansprüche**

1. Verbindung der Formel:

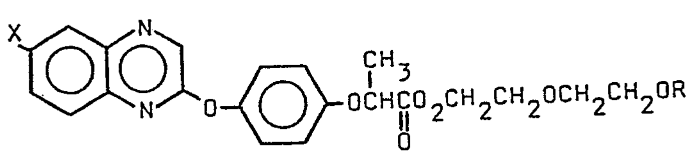

worin

X C, F oder Br ist; und

R $CH_3$ oder $CH_2CH_3$ ist;

ausgenommen Gemische von 2-Ethoxyethoxyethyl-2-[4-(6-chlorchinoxalin-2-yloxy)-phenoxy] - propanoat mit einem wesentlichen Anteil des entsprechenden 7-Chlorisomeren.

2. Verbindung nach Anspruch 1, nämlich 2-Ethoxyethoxyethyl-2-[4-(6-chlorchinoxalin-2-yloxy) - phenoxy] - propanoat, ausgenommen Gemische davon mit einem wesentlichen Anteil des entsprechenden 7-Chlorisomeren.

3. Verbindung nach Anspruch 1, nämlich 2-Methoxyethoxyethyl-2-[4-(6-chlor-2-chinoxalinyloxy) - phenoxy] - propanoat.

4. Verbindung nach Anspruch 1, nämlich 2-Methoxyethoxyethyl-2-[4-(6-fluor-2-chinoxalinyloxy) - phenoxy] - propanoat.

5. Zusammensetzung zur Steuerung unerwünschter Vegetation, umfassend eine wirksame Menge einer herbiziden Verbindung und mindestens eines von (a) einem oberflächenaktiven Mittel und (b) einem festen oder flüssigen Verdünnungsmittel, dadurch gekennzeichnet, dass die herbizide Verbindung eine Verbindung gemäss einem der Ansprüche 1 bis 4 umfasst.

6. Verfahren zur selektiven Steuerung unerwünschter Vegetation in Anwesenheit von Nutzpflanzen durch Auftrag auf den zu schützenden Ort von einer wirksamen Menge einer herbiziden Verbindung, dadurch gekennzeichnet, dass die herbizide Verbindung eine Verbindung gemäss einem der Ansprüche 1 bis 4 umfasst.

7. Verfahren nach Anspruch 6, bei dem die Nutzpflanzen Reis im Reisfeld sind.

8. Verfahren nach Anspruch 7, bei dem die Verbindung in einer Menge von 2 bis 20 g/ha aufgetragen wird.

9. Verfahren nach Anspruch 6, 7 oder 8, bei dem die Verbindung nach Anspruch 2 aufgetragen wird.

10. Verfahren zur Herstellung einer Verbindung nach Anspruch 1, welches umfasst:

(a) den Kontakt eines Alkalisalzes einer Verbindung der Formel

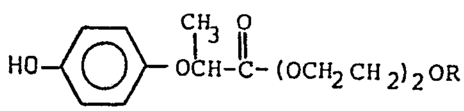

mit einem 2-Chlor-6-halogenchinoxalin der Formel

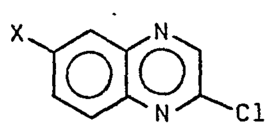

worin R und X wie in Anspruch 1 definiert sind; oder

(b) die Reaktion eines Säurechlorids der Formel

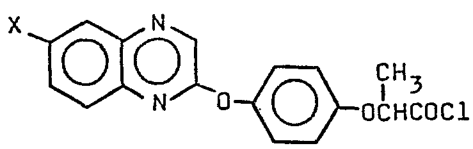

worin X wie in Anspruch 1 definiert ist, mit 2-(2-Methoxyethoxy)-ethanol oder 2-(2-Ethoxyethoxy) - ethanol, in Anwesenheit eines Säureakzeptors; oder

(c) Reaktion eines 6-Halogen-2-(4-hydroxyphenoxy)-chinoxalins der Formel

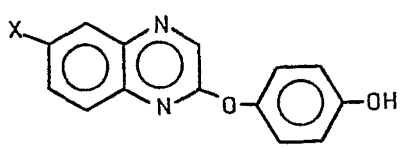

mit einem Bromester der Formel

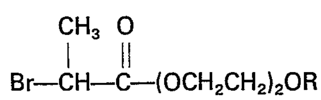

in Anwesenheit eines Säureakzeptors, worin X und R wie in Anspruch 1 definiert sind.

**Revendications**

1. Un composé de la formule:

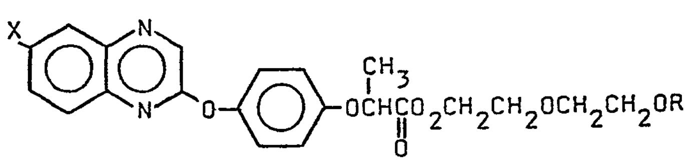

où

X est Cl, F ou Br; et

R est $CH_3$ ou $CH_2CH_3$;

à l'exclusion de mélanges de 2-[4-(6-chloroquinoxaline-2-yloxy)phénoxy)-propanoate de 2-éthoxyéthoxyéthyle et d'une proportion notable de l'isomère 7-chloro correspondant.

2. Le composé selon la revendication 1, qui est le 2-[4-(6-chloroquinoxaline-2-yloxy)phénoxy] - propanoate de 2-éthoxyéthoxyéthyle, à l'exclusion des mélanges de celui-ci et d'une proportion notable de l'isomère 7-chloro correspondant.

3. Le composé selon la revendication 1, qui est le 2-[4-(6-chloro-2-quinoxalinyloxy) - phénoxy] - propanoate de 2-méthoxyéthoxyéthyle.

4. Le composé selon la revendication 1, qui est le 2-[4-(6-fluoro-2-quinoxalinyloxy) - phénoxy] - propanoate de 2-méthoxyéthoxyéthyle.

5. Une composition pour lutter contre la végétation indésirable comprenant une quantité efficace d'un composé herbicide et au moins un des ingrédients suivants: (a) un agent tensio-actif et (b) un diluant solide ou liquide, caractérisée en ce que le composé herbicide comprend un composé selon l'une quelconque des revendications 1 à 4.

6. Un procédé pour lutter sélectivement contre la végétation indésirable en présence de plantes cultivées, par application, au lieu à protéger, d'une quantité efficace d'un composé herbicide, caractérisé en ce que le composé herbicide comprend un composé selon l'une quelconque des revendications 1 à 4.

7. Un procédé selon la revendication 6, dans lequel les plantes cultivées sont du riz.

8. Un procédé selon la revendication 7, dans lequel on applique le composé à une dose de 2 à 20 g/ha.

9. Un procédé selon la revendication 6, 7 ou 8, dans lequel on applique le composé de la revendication 2.

10. Un procédé de préparation d'un composé selon la revendication 1, qui consiste:

(a) à mettre en contact un sel de métal alcalin d'un composé de formula:

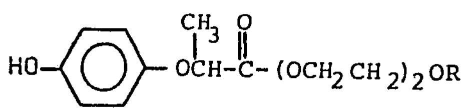

avec une 2-chloro-6-halogénoquinoxaline de formule:

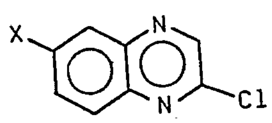

dans laquelle R et X sont tels que définis à la revendication 1; ou

(b) à faire réagir un chlorure d'acide de formule:

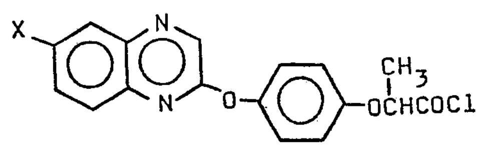

dans laquelle X est tel que défini à la revendication 1, avec du 2-(2-méthoxyéthoxy)-éthanol ou du 2 - (2 - éthoxyéthoxy) - éthanol en présence d'un accepteur d'acide; ou

(c) à faire réagir une 6-halogéno-2-(4-hydroxyphénoxy)-quinoxaline de formule:

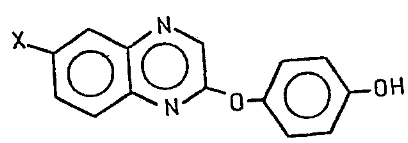

sur un ester bromé de formule:

$CH_3$ O
| ||
Br—CH—C—$(OCH_2CH_2)_2OR$

en présence d'un accepteur d'acide, où X et R sont tels que définis à la revendication 1.